# EUROPEAN PATENT SPECIFICATION

(11) **EP 2 512 352 B1**
(45) Date of publication and mention of the grant of the patent: **27.09.2017**
(21) Application number: 10842570.3
(22) Date of filing: 15.12.2010
(51) Int. Cl.: A61B 17/12, A61L 31/04, A61L 27/14, A61M 25/01

(54) **MULTI-FIBER SHAPE MEMORY DEVICE**
FORMSPEICHERNDE MEHRFASERVORRICHTUNG
DISPOSITIF MULTIFIBRE À MÉMOIRE DE FORME

(30) Priority: 16.12.2009 US 286955 P
(43) Date of publication of application: 24.10.2012
(73) Proprietor: Endoshape, Inc., Boulder, CO 80301 (US); The Regents of the University of Colorado, A Body Corporate, Denver, CO 80203 (US)
(72) Inventor: TROMMETER, Julie, Lafayette Colorado 80026 (US); CASTLEBERRY, Jeff, Longmont, Colorado 80503 (US); ALDRICH, Bill, Napa California 94581 (US); SHANDAS, Robin, Boulder Colorado 80301 (US); FOGELBERG, Jim, Boulder Colorado 80302 (US); LYONS, Mike, Boulder Colorado 80303 (US)
(74) Representative: Hanna Moore + Curley
(86) International application number: PCT/US2010/060598
(87) International publication number: WO 2011/084536

(56) References cited:
- EP-A2- 2 098 174
- WO-A1-00/10469
- WO-A2-2010/115076
- US-A1- 2004 193 246
- US-A1- 2005 021 074
- US-A1- 2005 038 460
- US-A1- 2007 016 233
- US-A1- 2009 275 974

## Description

### TECHNICAL FIELD

The invention relates generally to implantable devices for interventional therapeutic treatment, and more particularly concerns an endoluminally delivered device for cerebral aneurysm isolation and repair.

### BACKGROUND

Interventional Neuroradiology (INR) is a medical discipline expanding minimally invasive treatments for intracranial vascular defects and vascular malformations while avoiding the cost and burden of open surgery. INR treatments include treating cerebral vascular malformations such as cerebral aneurysms, a bulging and weakening of the vessel wall; and arteriovenous malformations (AVMs) wherein the artery and vein are connected. Vasoocclusive (V-o) devices are typically used to isolate and/or fill the defect. Other INR procedures include occlusion of arteriovenous fistulae (AVF), parent vessel sacrifice (PVS), and tumor indications among other conditions.

INR clinicians utilize various imaging modalities (primarily fluoroscopy) along with percutaneous (vascular access) guide and delivery catheters to routinely conduct, for example, vascular "stenting" to maintain patency of a diseased vessel lumen or provide support of at the neck of an aneurysm to retain a second device for aneurysm repair, and/or aneurysm embolization to isolate a vascular aneurysm's weakened vessel wall from blood flow and blood pressure.

Cerebral aneurysms tend to occur in anterior and posterior communicating arteries, and a middle cerebral artery. The majority of these aneurysms are located at either curvature in the vessels or at bifurcations of these vessels. These defects can be detected prior to rupture through related symptoms (e.g., affected vision, headaches, etc). However, cerebral aneurysms are most often diagnosed by the rupture and subarachnoid bleeding within the brain, and in many instances, leading to debilitating stroke and/or death.

Currently, cerebral aneurysms are commonly treated in open surgical procedures where the diseased vessel segment is clipped across the base of the aneurysm. While considered to be an effective surgical technique, particularly considering an alternative which may be a ruptured or re-bleed of a cerebral aneurysm, conventional neurosurgery suffers from a number of disadvantages. The procedure is highly invasive, requiring sawing through the bony cranium; it requires experienced surgeons and well- equipped surgical facilities; and surgical cerebral aneurysm repair has a relatively high mortality and morbidity rate of about 2% to 10%.

Alternatively, patients are referred by the neurosurgeon to INR physicians for INR coiling or aneurysm isolation (both pre-ruptured and ruptured defects) when the site is inaccessible for surgical treatment or the neurosurgeon believes the aneurysm characteristics may be a good candidate for INR coiling. Historically, some INR coiled patients have required retreatment due to a reoccurrence of the aneurysm believed to be caused by insufficient packing of the coil material in the aneurysm sack. As coils are introduced into the aneurysm by a very small delivery catheter, the coils form a "coil pack" to isolate the weakened vessel wall from the primary vessel's blood flow and pressure. Voids in the coil pack, developed either during the procedure or post operatively, cause channels for blood flow and pressure to reach the weakened vessel wall resulting in a continuation of the risky disease state and potential for aneurysm rupture. Successful isolation (either via surgical clipping or INR coiling) results in an endothelial cell to endothelial cell closure of the aneurysm and therefore a cure for the disease.

As such, the effectiveness of INR coiling to achieve cerebral aneurysm repair is clinically perceived, in part, by the packing factor achieved by the vaso-occlusive ("v-o") device. Packing factor is a measure of the aneurysm's sack volume displaced by the v-o device. For a given size aneurysm, higher packing factors measured during the procedure are associated by clinicians with a lower incidence of reoccurrence and need for retreatment. Low packing factors allow the paths for blood flow and pressure to become re-established and stress is again exerted on the weakened vessel wall. For small aneurysms (<10mm diameter), packing factors of 40% are considered very good with metal coils. Other devices, utilizing non-metal materials have attempted to increase the packing factor above this level with limited success.

The v-o devices can take a variety of configurations, and are generally formed of one or more elements that are larger in the deployed configuration than when they are within the delivery catheter prior to placement. One widely used v-o device is a helical wire coil having a deployed configuration which may be dimensioned to engage the walls of the anatomical space, e.g., vessel. One anatomically shaped v-o device that forms itself into a shape of an anatomical cavity such as an aneurysm and is made of a pre-formed strand of flexible material that can be a nickel-titanium alloy is known. That v-o device comprises one or more members wound to form a generally spherical or ovoid shape in a relaxed state. The members can be a helically wound coil or a co-woven braid formed of a biocompatible material, and the device is sized and shaped to fit within a vascular cavity or vessel, such as for treatment of an aneurysm or a fistula. The v-o member can be first helically wound or braided in a generally linear fashion, then wound around an appropriately shaped mandrel or form, and heat treated to retain the shape after removal from the heating form. The primary shape is a coil of wire that is then formed into a more complex secondary shape. The primary coil comprises a wound resilient alloy wire formed as a straight coil, on which a bent secondary shape with coiled ends is imposed. There are many other variations of metal embolic coils known including those with offset helical and twisted shapes having multiple axially offset longitudinal or focal axes with a secondary shape with coiled ends and a middle loop. A stretch-resistant v-o coil is also known that is formed from a helically wound primary coil and a stretch resistant member, that can also have a secondary shape with coiled ends and a middle loop, and an embolization coil having a single closed loop. Highly flexible coils with secondary shape are also known that form an occlusive implant that is sufficiently flexible that it can be damaged during handling and maintain that configuration. It has been found that single strands of small diameter nickel-titanium alloys, as well as other metal alloys, used to form metal v-o coils can be kinked if twisted and pulled as can occur during or after deployment from a catheter, especially if the doctor wishes to withdraw a partially deployed coil because it is somehow incorrect in size, shape or length to effect the desired repair.

Because a wire wound platinum coil form is a common construction for catheter guidewires and has been adapted for occlusive coils, significant IP effort has focused on key incremental features that define an acceptable product. Some metal coils are coated with a bioabsorbable polymer (PLGA, etc) as a means of enhancing blood/tissue interaction. Other coils are coated with a hydrogel to cause them to swell in-situ and provide a tighter coil pack. However, all of these products rely on an underlying metal coil design.

Regarding metal coils, micro-coils formed of very small diameter wire may be used in order to restrict, reinforce, or to occlude such small diameters areas of the vasculature. A variety of materials have been suggested for use in such micro-coils, including nickel-titanium alloys, copper, stainless steel, platinum, tungsten, various plastics or the like, each of which offers certain benefits in various applications. Although various materials are more or less kink resistant when nickel-titanium alloys are dimensioned into wire smaller than approximately 0.254 mm (0.010 inches) in diameter, they can have low yield strength and can kink more easily, thereby limiting the applications for such finely drawn wire in the fabrication of v-o devices. As a further limitation to such applications, nickel-titanium alloys are not radiopaque in small diameters, and a single nickel-titanium wire may need to be approximately 0.305 mm (0.012 inches) in diameter to be somewhat radiopaque. However, such a thickness of a single nickel-titanium wire would also be relatively stiff and possibly traumatic to the placement site, particularly if used for treatment of delicate and already damaged areas of the small diameter vasculature such as an aneurysm in an artery or vein in the brain.

Recently, polymer coils have become available which provide a continuous single fiber that is delivered and trimmed to length. However, some polymer coils include specific limitations relying on delivery of a single fiber and require a specific catheter to achieve the cutting/trimming of the single fiber. Trimming/cutting during surgery may also increase procedure time and require a specialized tool to deliver and perform the procedure. Further, traditional polymers that are not shape memory polymers cannot provide suitable "shape certainty" after storage in a constrained or stressed condition for extended periods. Traditional polymers suffer from "creep" resulting in a loss of shape memory when deployed from a constrained package. Traditional polymer devices do not exhibit shape certainty as a predictable, precise shape after deployment.

Also, current metal and polymer coils utilize resilient materials operating within simple elastic limits to maintain the unique coiled sample post deployment (if at all) while the device is being held in a straight configuration inside of a constrained package such as a coil holder (tube/hub device) that allows easy physician loading into the proximal end of the delivery catheter. Again, these devices suffer performance limitations and high cost constraints due to the underlying materials of construction and the configuration of deployment-a single fiber delivery.

In U.S. Patent Application Publication No. 2008/0319527 (WO2009/002403) entitled "Shaped multi-durometer filler," an "aneurysm filler" is described as a single fiber that incorporates a "transport shape" and a "three dimensional shape which is manifest when the main body is deployed in an aneurysm." However, the aneurysm filler material does not have a specific predetermined shape, nor does it present shape certainty of reproducing that predetermined shape upon delivery of the single fiber material into the aneurysm. In this way, the aneurysm filler single fiber material has the potential to come into sharp contact with the fragile diseased vessel wall of an aneurysm, increasing the potential for rupture or injury.

Delivery of such a coil in the treatment of aneurysms or other types of arteriovenous malformations can be accomplished by a variety of means, including via a catheter in which a single device is pushed through the catheter by a pusher attached to the coil. The coil passes through the lumen of the catheter in a linear shape and takes on a complex shape as it was originally formed after being deployed into the area of interest, such as an aneurysm. Varieties of precision detachment mechanisms to release the single coil from a pusher have been developed and are known.

To complete an occlusion procedure, the physician must sequentially reload the catheter with several individual coils until he/she has determined the occlusion is sufficient. Likewise, to fill isolate an aneurysm, the physician must sequentially reload the catheter with several individuals coils, experiencing some trepidation of getting the coil positioned and detached correctly each time. This physician typically determines whether sufficient coils have been deployed by assessing the level of occlusion of the vessel flow or by evaluating the density of the coil packed into the aneurysm sack (e.g., coil pack), both performed by using contrast media with typical medical imaging techniques. This "place and assess" method can extend the medical procedure time, cost and also can increase the imaging exposure (e.g., radiation exposure) to both the patient and the physician.

The information included in this Background section of the specification, including any references cited herein and any description or discussion thereof, is included for technical reference purposes only and is not to be regarded subject matter by which the scope of invention is to be bound.

WO0010469 (A1) discloses a vasoocclusive coil which has a primary coil configuration with a helical loop at least one end. The terminal helical loop can have a J-shaped configuration, preferably with a loop diameter of about 2 mm. The coil is preferably provided with helical loops at both ends, with helical loop at the proximal and distal ends of the coil acting as an anchor to prevent the coil from coming free from the location being treated and escaping into the vasculature.

In US2009275974 (A1) devices and methods for treatment of a patient's vasculature with some embodiments configured for delivery with a microcatheter for treatment of the cerebral vasculature of a patient are described. Some embodiments may include a permeable shell configured to occlude blood flow therethrough.

WO2010115076 (A2) describes shape memory materials (SMM) which are formed as coil-shaped vascular occlusion devices upon deployment. Shape memory polymer (SMP) materials are tailored through formulation for specific mechanical behavior of the coils. Concurrent coil diameter changes enhance the relative change in stiffness along the length of the coil. Interconnecting structures are formed on ends of elongated members in the pre-deployment shape for multiple coil insertion capability within an introducer. Channels are formed in pre-deployment shape, elongate members that allow access for injection of imaging contrast agent or concurrent placement of instruments. A single SMM occlusive device transforms into multiple, smaller diameter coils in the deployed state to generate a complex occlusive structure. A SMM occlusive device has a collapsed fabric component attached to and extending along a sidewall during storage and insertion, and then deploys as a coil to form a single- or multiple-layer occlusive fabric surface within a center of the coil.

EP2098174 (A2) discloses a system that includes a catheter including a distal portion for location at least partially in an aneurysm, the catheter including a proximal portion, the catheter to transport a biocompatible polymeric strand to an aneurysm, a biocompatible polymeric strand sized to slide through the catheter and a heated excise collar coupled to the distal portion of the catheter, the heated excise collar to provide radial heat from the catheter inward to melt and excise the biocompatible polymeric strand.

### SUMMARY

The present teaching provides a shape memory coiling device as detailed in claim 1. Also provided is a method according to claim 7. Advantageous features are provided in the dependent claims.

In one implementation, a shape memory coiling device is provided for occluding a vascular target. The device may be formed of a shape memory polymer material. The shape memory material may be formed in a formation state as a plurality of coiled members and may have a specific shape and stiffness which is then constrained into a pre-deployed state as a plurality of elongate members configured for delivery through a delivery device wherein the plurality of elongate members are aligned axially with respect to each other and the delivery device. The shape memory material, in a pre-deployed state, may also exhibit stiffness that enhances the ability to deliver the shape memory material through the delivery device. In some embodiments, the shape memory material may be formed in an intra-deployed state such that at least one of the plurality of elongate members will anchor the device before filling the vascular target and becoming a coiled member. In some embodiments, the shape memory material may be formed in an intra-deployed state such that all of the elongate members coil upon delivery through a delivery device to form a non-anchored coil pack that enlarges with the addition of more material coming into contact with the anatomical space upon delivery of sufficient material. The shape memory material may be formed in a deployed state as a plurality of coiled members. In some embodiments, the vascular target is an aneurysm. In some embodiments, the plurality of coiled members in the formation state is the same plurality of coiled members as in the deployed state.

In another implementation, a shape memory coiling device for occluding a vascular target may be formed of a shape memory polymer material. The shape memory material may be formed in a pre-deployed state as a plurality of elongate members configured for delivery through a delivery device wherein the plurality of shape memory material elongate members are grouped or connected together at a midpoint of each individual member, each individual member having at least two zones of material with different material properties, such as modulus, or Tg. The shape memory material may be formed in an intra-deployed state such that at least one of the plurality of elongate members will anchor the device before filling the vascular target and becoming a coiled member. The shape memory material may be formed in a deployed state as a plurality of coiled members. In some embodiments, the vascular target is an aneurysm. In some embodiments, the at least two zones of material are aligned relative to each individual elongate member. In some embodiments, the at least two zones of material are not aligned relative to each individual elongate member. In some embodiments, each individual member of the plurality of elongate members has the same length. In some embodiments, each individual member of the plurality of elongate members has a different length.

In a further implementation, a shape memory coiling device for occluding a vascular target may be formed of a shape memory polymer material. The shape memory material may be formed in a deployed state of specific shape and stiffness which is then constrained into a pre-deployed state as a plurality of elongate members configured for delivery through a delivery device wherein the plurality of shape memory material elongate members are grouped or connected together at a midpoint of each individual member and folded to be configured in a constrained pre-deployed state. The shape memory material may be formed in an intra-deployed state such that all of the elongate members coil upon delivery through a delivery device to form a coil pack within the vascular target that enlarges with the addition of more material. The shape memory material may be formed in a deployed state such that the midpoint fold of the members unfolds when deployed to avoid loose ends of the elongate members from being exposed outside of the coil pack and to provide a bridgework of the elongate members across the vascular target opening at the conclusion of delivery of the coiling device. In some embodiments, the vascular target is an aneurysm.

In another implementation, a shape memory coiling device for occluding a vascular target may include a shape memory polymer material. The shape memory material may be formed in a deployed state of specific shape and stiffness which is then constrained into a pre-deployed state as a plurality of elongate members configured for delivery through a delivery device wherein the plurality of shape memory material elongate members are grouped or connected together at a proximal endpoint of each individual member to be configured in a constrained pre-deployed state. The shape memory material may be formed such that all of the elongate members curl upon delivery through a delivery device to form a coil pack within the vascular target that enlarges with the addition of more material thereby providing a bridgework of the elongate members across the vascular target opening at the conclusion of delivery of the coiling device.

In another implementation, a method of manufacturing a shape memory coiling device for occluding a vascular target is disclosed. A delivery device having a pusher mechanism with a detachment feature is provided. A shape memory polymer material formed in a pre-deployed state as a plurality of elongate members is also provided that is configured for delivery through the delivery device. Each individual member has at least two zones of material with different Tg. A retaining loop across a midpoint of each individual member of the plurality of elongate members is provided. The elongate members are formed about the retaining loop to form a proximal end and a distal end of the plurality of elongate members. The proximal end of the plurality of elongate members is operably attached to the detachment feature of the pusher mechanism of the delivery device.

This Summary is provided to introduce a selection of concepts in a simplified form that are further described below in the Detailed Description. This Summary is not intended to identify key features or essential features of the claimed subject matter, nor is it intended to be used to limit the scope of the claimed subject matter. Other features, details, utilities, and advantages of the present invention will be apparent from the following more particular written description of various embodiments of the invention as further illustrated in the accompanying drawings and defined in the appended claims.

### BRIEF DESCRIPTION OF THE DRAWINGS

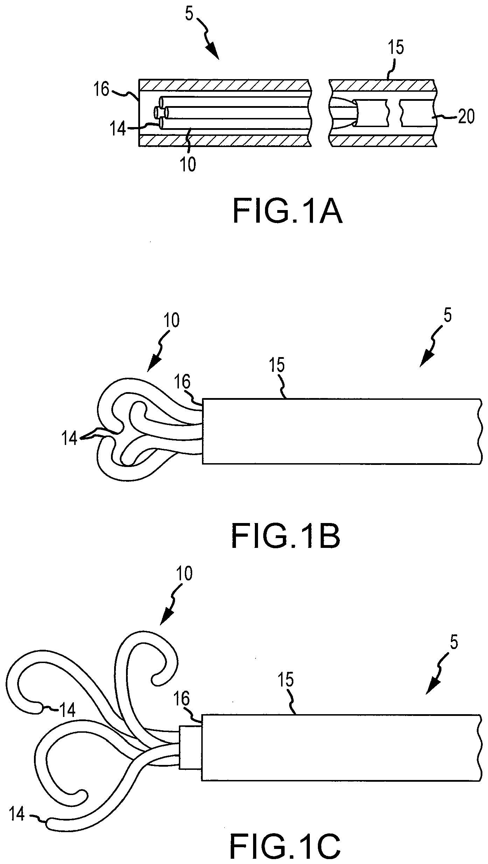

FIG. 1A is a cross-section view of a catheter delivering a shape memory coil device in a pre-deployed state formed with a collection of coil tendrils attached to a base.
FIG. 1B is a side elevation view of the coil device of FIG. 1A deploying from a catheter and forming a complex occlusive coil structure.
FIG. 1C is a side elevation view of the coil device of FIG. 1A fully deployed, but not yet released from a delivery catheter.
FIG. 1D-1 is an isometric, side elevation view of another embodiment of the coil device , wherein the coils are shown partially deployed from a catheter.
FIG. 1D-2 is an isometric, side elevation view of the coil device of FIG. 1D-1, wherein the coils are shown fully deployed, but not yet released, from a catheter. The coils are depicted to highlight differences in structure, in this case different diameters and shapes.
FIG. 2A depicts the coil device of FIG. 1A deploying into a simulated, non-symmetrical basalier aneurysm.
FIG. 2B depicts the coil device of FIG. 2A wherein ends of the coil device are shown engaging at least one wall of the aneurysm.
FIG. 2C depicts the coil device of FIG. 2A wherein the coil device continues to be deployed and the device continues to engage at least one wall of the aneurysm.
FIG. 2D depicts the coil device of FIG. 2A shown in a completely deployed state wherein a proximal end of the device is shown transversing an opening of the aneurysm.
FIG. 3A depicts another embodiment of a coil device, wherein the coil device is shown in its formation state with elongate curled members (post deployed shape) aligned at ends and center.
FIG. 3B depicts the coil device of FIG. 3A, wherein the coil device is shown in its formation state with elongate members manipulated as straight members (pre-deployed or stored shape) aligned at ends and center.
FIG. 3C depicts the coil device of FIG. 3B, wherein a retaining loop is disposed about a midpoint of a plurality of elongate members of the coil device.
FIG. 3D depicts the coil device of FIG. 3C, wherein the elongate members of the device are shown in a pre-deployed state restrained at a midpoint by a retaining loop.
FIG. 3E depicts the coil device of FIG. 3D, wherein the coil device is shown within and deploying from a delivery catheter.

### DETAILED DESCRIPTION

The target anatomy for vascular malformations (e.g., cerebral aneurysms, AVMs, etc) are pathologic structures and present significant anatomical variability. Therefore, a clinically acceptable device is flexible and adaptive to the structure it is filling without inducing significant pressure on the friable, fragile diseased vessel wall. As indicated, metal embolic coils come in a variety of single devices, of differing shapes and sizes, defined for specific purposes: framing, filling and finishing. Typically these are made from platinum metal wire, with various precision features for detachment. However, metal wire may limit not only the clinical performance of such devices but also induces high manufacturing costs.

As an alternative to present metal coil designs, a shape memory coil device 5 is disclosed herein, which may have a plurality of fibers or elongate members 10 that make up the coils. The device 5 may utilize shape memory polymers (SMP) for the coil material. Although the disclosure describes the shape memory coil device 5 in terms of SMP materials, it should be understood that other shape memory materials may be utilized, including, for example, a shape memory metal alloy or other shape memory material.

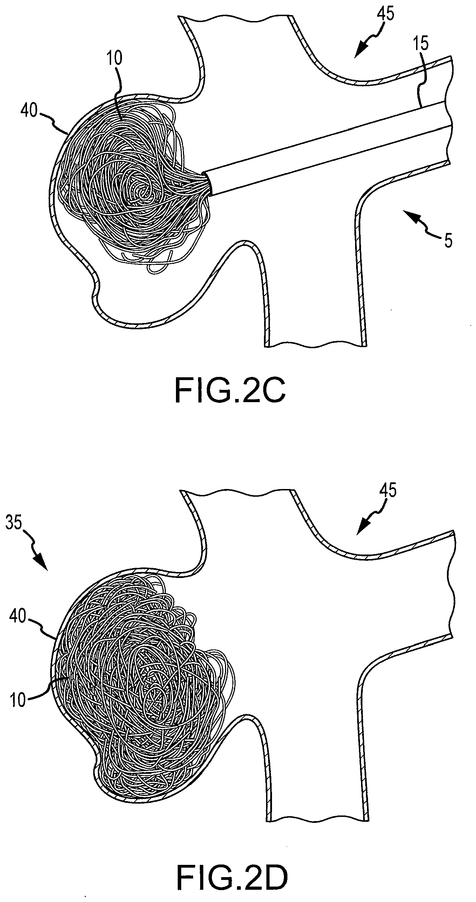

As can be generally understood with reference to FIGS. 1A-2D, this disclosure describes a multi-fiber occlusive device 5 wherein multiple, very small coils 10 (fibers, tendrils, or similar structures) are delivered simultaneously to form an equivalent coil pack (for example, a coil pack 35 as described with reference to Fig. 2D) that would otherwise be formed by the accumulation of individual coils delivered sequentially. This simultaneous delivery and rapid formation of a coil pack provides key benefits to an aneurysm repair device or vascular occlusion device. This configuration also provides a flexible and adaptive structure for filling these aneurysm sacks or other vascular structures.

The multi-fiber configuration disclosed herein may be used, for example, for filling a cerebral aneurysm or AV malformation, although for ease of the reader, the device is discussed in terms of a cerebral aneurysm. However, it is understood that the device 5 may be utilized for other vascular malformations, such as ateriovenous malformation (AVM), or ateriovenous fistulae (AVF), parent vessel sacrifice (PVS), and tumor indications, among other uses. Thus, the devices and methods disclosed herein may also be used for occlusion of other vascular target sites. These target sites may also be larger vessels, such as vessels between approximately 3mm and approximately 10mm in diameter.

For a discussion of the device 5, reference is now made to FIGS. 1A-2D. In use, and as shown in FIG. 1A, the device 5 is loaded into a catheter or other delivery device 15 in a non-expanded state. Once the surgeon has placed the delivery device 15 into the proper location, the device 5 may be pushed by a "pusher" 20 out of the delivery device 15, as indicated by FIG. 1B and into its deployed or expanded state, as indicated in FIG. 1C. The straightened coil device 5 (in its non-expanded shape) is deployed by advancing it down the catheter/delivery device 15, using a pusher 20, and pushing it out the distal end 16 of the delivery device 15 at the target occlusion site. A detachment device uniquely configured for holding and releasing the coil device 5 may be employed to control the release of the coil device by the physician.

As the fibers 10 deploy and regain their memory shape, the fibers expand, coil, and form a coil pack 35 (e.g. as illustrated in FIG. 2D). The fibers 10 may push against the vessel wall 40 initially, during deployment, or near the end of deployment depending on the embodiment and depending upon the formulation of the SMP material used, which can be varied to, *inter alia,* increase or decrease the deployment time for the material. The fiber ends are biased to be positioned within the aneurysm or other vascular structure in need of occlusion.

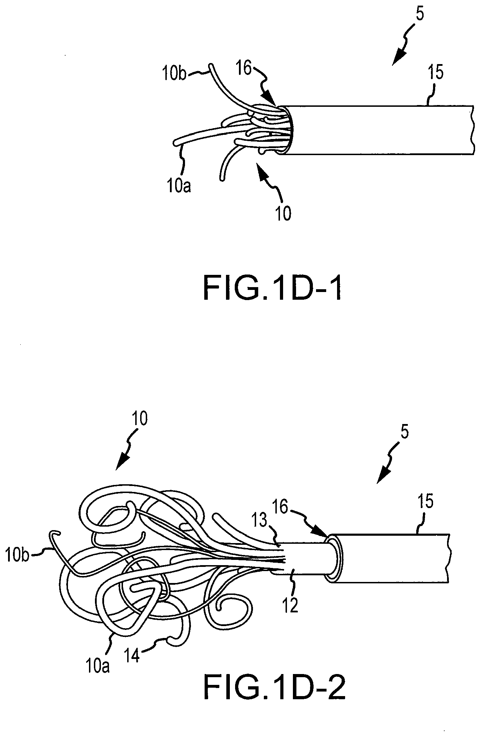

In some embodiments, as illustrated in FIGS. 1D-1 and 1D-2, the multiple fibers 10, in a deployed state, may have complex curl shapes, multiple size curls and/or multiple size fibers with respect to either diameter, length, or both. In various embodiments, some fibers 10a may be produced larger in diameter than others to be stiffer and thereby assist in anchoring the coil pack. Other fibers 10b for use in the same device 5 may be produced softer, i.e., of a smaller diameter, to assist in quickly filling the pack and minimizing the coil pack length. Stiffness and softness of different fibers may also be achieved by varying the SMP formula as between different fibers to vary the material properties.

In one exemplary embodiment, the device 5 may be formed by 9 individual fibers 10 (members), all from the same material. In this embodiment, three are 0.3048mm (0.012") in diameter and curled at a radius of 4mm, three are 0.3048mm (0.012") in diameter and curled at a radius of 3mm, and three are 0.381 mm (0.015") in diameter and curled at a radius of 2mm. In this embodiment, all the coils are of a non-helical curl shape-the curl is instead more like a cloverleaf. Other curl shapes may be used. In a pre-deployed arrangement of the formation state, laid flat, each fiber is 9cm long. As indicated in Fig. 1D-2, the fibers are grouped together and the proximal ends 13 of the fibers 10 are bonded by a connection member 12, such as a small plug form (e.g., in one embodiment about 5mm long). The forming tube may be removed after curing such that the connection member 12 operably connects the proximal ends 13 together. In some disclosed examples not being part of the invention, the fibers may be connected (or attached) at both a proximal end 13 and a distal end 14 of the fibers 10.

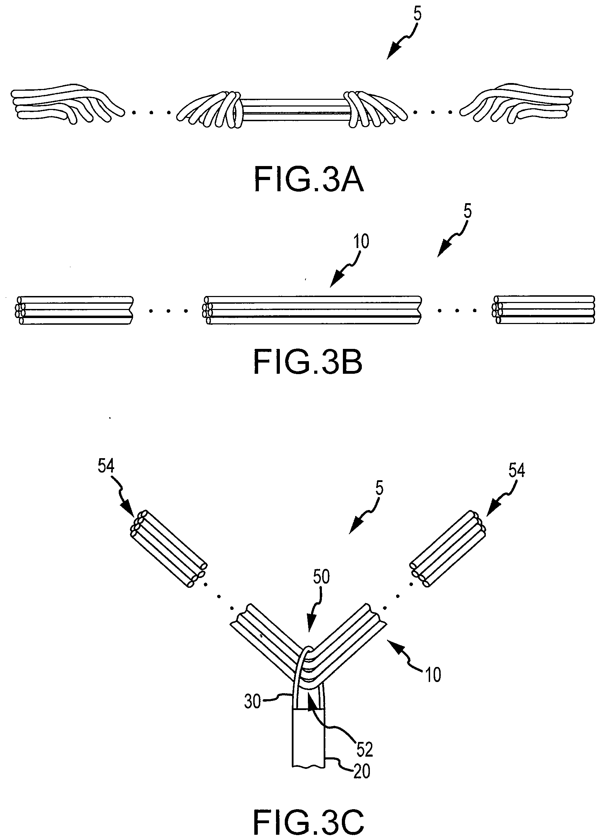

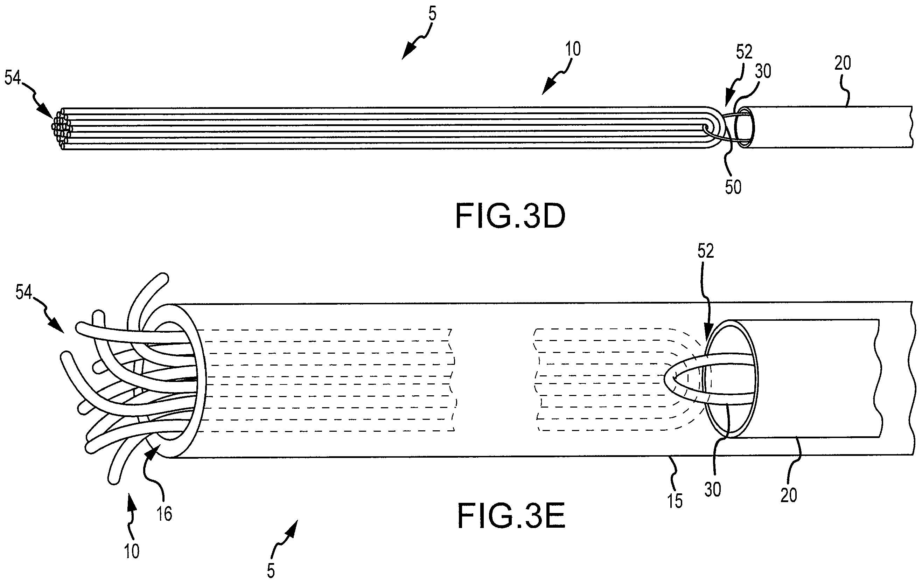

As described in more detail below, coiling generally employs coils 10 that will retain their unique coil shape after deployment due to elastic recovery in the material, subsequent to significant storage time being held in a straight coil introducer. Coils 10 enter a delivery catheter 15 in the straight (pre-deployed) form and are advanced down the delivery catheter 15 using a guidewire "pusher" 20. Coils for cerebral aneurysm repair may utilize dedicated pushers 20 that are affixed to the coil 10 with a reliable detachment feature 30 (e.g. a retaining loop 30 or other appropriate device as discussed in more detail below and with reference to FIGS. 3C-3E) such that the coil can be released within the aneurysm at the physician's discretion.

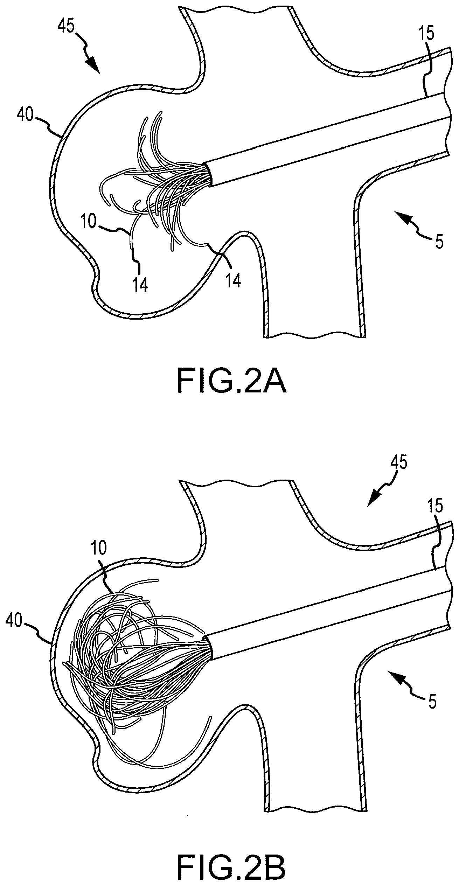

FIGS. 2A-2D depict one embodiment of the coil device 5 within a simulated, non-symmetrical aneurysm 45. As shown in FIG. 2A, the device 5 is in a pre-deployed or constrained state and is being advanced down a delivery catheter 15. The fibers of the coil device 5 are contained within the catheter 15. As the distal ends 14 of the fibers 10 of the coil device 5 extend beyond the distal end 16 of the catheter or other delivery device 15, the fibers 10 expand and begin to coil. In this way, the loose ends of the fibers 10 are contained within the aneurysm sack 45. The fibers 10 continue to advance and contact at least one wall 40 of the aneurysm 45, as shown in FIGS. 2B and 2C. As can be understood from FIG. 2C, as the fibers 10 continue to advance, the fibers 10 begin to form a coil mass 35, or coil pack, before the multi-fiber coil device 5 is fully deployed from the delivery device 15.

As shown in FIG. 2D, once the multi-fibered coil device 5 has fully exited the delivery device 15, the coil device 5 will generally fill the aneurysm 45 or a portion of the aneurysm as determined by the physician. The proximal end 13, 52 of the multi-fibered device 5 may unfold and have a memory configuration designed to traverse, or bridge, the opening of the aneurysm, thereby mitigating concerns about proximal ends 13, 52 loosely falling into the parent vessel. Thus, the intended result is achieved when the sack 45 is filled to isolate the diseased vessel wall from blood flow and blood pressure. Isolation occurs in part from the volume displacement by the coils in concert with palette formation and thrombus filling the interstitial spaces resulting in complete isolation of the aneurysm sack from the parent vessel.

The nonmetallic, thermo-sensitive, shape memory materials provide key benefits to an aneurysm repair device. The shape memory coil device 5 may also be a multi-fiber device 5 wherein multiple, very small coils (fibers) 10 are delivered simultaneously to form an equivalent coil pack that would otherwise be formed by the accumulation of individual coils delivered sequentially. This simultaneous delivery and rapid formation of a coil pack provides key benefits to an aneurysm repair device.

The device disclosed herein includes fibers that may be configured from nonmetallic, SMP materials, which are uniquely formulated to provide radiopacity to facilitate imaging under standard fluoroscopy while allowing use of CT scan and MR imaging methods, not achievable with metal coils. SMP material properties can be tailored, through formulation, for specific mechanical behavior of each fiber. This includes, but is not limited to: zones of differing post deployment; modulus or stiffness of the material; fiber diameter changes, including concurrent diameter changes with each zone that can enhance the relative change in stiffness along the length of each fiber; and zones of differing Tg's along the length to effect different rates of shape change and effectively time the deployment of specific fiber features. The acrylate- and/or thiol-based Shape Memory Polymers exhibit exceptional (∼99%) "shape certainty" (predictable and precise material properties and dimensions for pre- and post-deployment), which enables the distinct behaviors of the fibers to enhance the functionality and utility of the device.

In some embodiments, polyester fibers may be incorporated into the SMP fibers or laid up in parallel with the SMP fibers during assembly for enhanced thrombogenicity. The pre-deployed behavior of the stored shape form enhances pushability of the device through a very small delivery catheter within a tortuous path. The post-deployed behavior (i.e., the deployed shape form) enhances the aneurysm sack-filling capabilities while minimizing risks associated with perforation or rupture. These two definitive behavior/shapes provide significant feature advantages over traditional polymer, elastomeric, metal or other flexible materials operating within a simple a material's elastic limit.

Post-deployed SMP material behavior can achieve a lower modulus resulting in softer fibers than achievable with metal coils or continuous traditional polymer coils. As such, more material can be packed within the aneurysm space without putting excessive pressure on the vessel wall. Therefore, the multi-fiber SMP aneurysm embolization device as disclosed herein can achieve higher packing factors in aneurysm repair than can be achieved with metal or continuous traditional polymer coils.

Further, the multi-fiber device 5 includes multiple, very small coils 10 (fibers) that are delivered simultaneously to form an equivalent coil pack that would otherwise be formed by the accumulation of individual coils delivered sequentially. This simultaneous delivery and rapid formation of a coil pack provides another benefit to the coil or multi-fiber embolization device disclosed herein.

In order to better describe the SMP coil and multi-fiber embolization device 5, it is useful to provide a background on shape memory polymers. SMPs demonstrate the phenomena of shape memory based on fabricating a segregated linear block co-polymer, typically of a cross-linked hard segment and a soft segment. The shape memory polymer generally is characterized by defining phases that result from glass transition temperature (Tg). Mechanical properties of the phases, stored shape (e.g., below the Tg) and the deployed shape (e.g., above the Tg) as well as setting the Tg can be tailored by adjusting the formulation through different weight percentages of the monomers and cross linker. (See Patent Cooperation Treaty application nos. PCT/US2007/065691 and PCT/US2006/060297). Shape memory polymers can be formulated for a Tg that allows use of an external heat source to initiate the phase change or a Tg that utilizes the body heat of the patient to initiate the phase change.

As SMP materials are formulated for use, other ingredients can be added to the formulation to induce specific properties or behavior. For example, Barium Methacrylate, Barium Acrylate, (dissolved in solution) or Tungsten or Bismuth titanate nanoparticles (held in suspension) or a combination of these or similar ingredients can be added to induce radio-opacity. Fibers or fabric from materials such as polyester can be added and positioned for surface exposure to induce thrombogenicity. Bioactive agents (e.g., fibroblast growth factor) and eluting pharmaceuticals (e.g., NSAID such as ibuprofen) can be integrated in the matrix of the material. The material can be treated with coatings such as hydrophilic coatings to reduce friction, or biodegradable coatings (e.g., polyglycolic acid) to induce a desired tissue response.

The implementation of SMP materials enables a multiple fiber aneurysm or other vascular malformation embolic (repair) device in unique ways that cannot be reproduced in metal or non-SMP materials. The coil device disclosed herein provides benefits including the following: reduced procedure time, reduced procedure cost, improved ease of use and improved occlusive outcome through multiple elements. In addition, using a shape memory polymer formulation in a multiple fiber configuration provides for several specific device advantages and/or benefits, including, but not limited to the following:
A) Thermomechanical response causes shape change from the formation state (i.e., that state in which the coiled members are formed) into a pre-deployed state, having straighter shape and higher stiffness that enhances push-ability and minimizes column buckling for delivery through a long and thin delivery catheter to a post-deployed, curled shape and softness within the aneurysm that enhances forming a coil mass while reducing the risk of aneurysm rupture/vessel wall puncture. In some embodiments, the plurality of coiled members in the formation state is the same plurality of coiled members as in the deployed state.
B) An intra-deployed rate of shape change effected by differing Tg (glass transition temperature) of the material in zones along the fiber allows timed functionality of the fiber feature to better assure anchoring prior to filling (which may or may not be used in some embodiments).
C) Very soft post deployment behavior allows for greater compaction between the fibers and results in higher packing factors than achievable with other materials. Higher packing factors are clinically perceived as helping to reduce the incidence of aneurysm reoccurrence.
D) Anchoring of the coil mass is enhanced in part due to the simultaneous delivery of multiple fibers. This allows the fibers to nest and quickly form a more stable mass than is otherwise developed by delivery of individual coils/fibers placed serially, wherein a partial coil mass may be very unstable and prone to migration into the parent vessel between delivery of sequential coils. Where the device is being used for vascular occlusion, immediate anchoring of the coil mass also prevents or hinders migration down the vessel.
E) Shape certainty of the deployed shape is achieved. Shape certainty refers to the property of the acrylate- and/or thiol-vinyl shape memory polymers that the final or deployed state shape is predictable and precise even after the device has been stored in a pre-deployed (straightened), constrained shape for long periods of time or with periods of elevated temperature. Shape certainty allows each fiber to be specifically defined by unique curl and arc configurations along each fiber, and between the multiple fibers in relation to each other and ensures that these shapes will be reformed with a high degree of accuracy (∼99%) upon deployment of the coil device. Shape certainly also provides, for example, controlled deployment of the fiber ends that are predisposed to stay within the aneurysm sack and not entrain back into the parent vessel, thereby reducing this risk during placement.
F) Controlled registration of each curl and arc aligned in adjacent fibers is realized such that the combined fibers can quickly and efficiently pack the aneurysm sack and provide suitable volume anchoring up against the aneurysm wall without generating excessive localized pressure that might otherwise cause a rupture.
G) Volume displacement of the deployed coil device may be accurately compared to the measured volume of the patient's aneurysm (via, e.g., preoperative CT scan) and therefore allow selection of the appropriate sized device, thereby minimizing the number of devices needed (e.g., one or two multi-fiber devices, versus 5 or 6 individual metal coils, or a specific or pre-determined length of polymer coil). In the case of vascular occlusion, the volume of the patient's vessel is estimated such that a device configured for a range of vessel sizes (e.g., 8-10mm diameter vessels) may be chosen.
H) Fibers, within the multiple fiber design, may exhibit unique material characteristics, e.g., different mechanical modulus between the adjacent fibers. Fibers of different diameters may also be utilized to achieve differing moduli between fibers and provide variable material volumes for deployment.
I) Fibers within the multiple fiber design may exhibit unique material characteristics, e.g., different mechanical modulus along each fiber in concert with the unique curl and arcs aligned in adjacent fibers.

Fibers of unique, non-metal material formulation may provide for suitable imaging under fluoroscopy as well as offer clinical use of other imaging modalities, e.g., CT scan and MRI, that otherwise could not be used with metal devices. These formulations include solutions of barium methacrylate or acrylate and suspensions of titanium, tungsten, or bismuth titanate nanoparticles.

An alternate implementation of a coiled device 5 is presented in FIGS. 3A-3E. In one embodiment, the elongated members 10 (fibers) are operably attached at a midpoint 50 by a retaining loop 30. As shown in FIG. 3A, a plurality of elongate members 10 of the device 5 may be laid out or axially aligned relative to each other in their deployed arrangement in the formation. As indicated in FIGS. 3B-3C, the elongate members 10 of FIG. 3A may then be "stretched out" or otherwise formed in their pre-deployed state for delivery through a delivery device 15. The delivery device 15 may have a pusher mechanism 20 with a detachment feature, such as the retaining loop 30. A midpoint 50 of the elongate members is located. A retaining member 30, such as a retaining loop, may be disposed about and operably connected to the midpoint 50. The retaining loop 30 may also be operably connected to a pusher mechanism 20 of a delivery device 15. The elongate members 10 may be folded about the retaining loop 30, thereby creating a proximal end 52 and a distal end 54 of the plurality of elongate members 10. As shown in FIGS. 3C-3E, the proximal end 52 of the elongate members 10 is operably attached to the retaining loop 30 and will be maintained in this constrained or pre-deployed state for delivery by a delivery device 15.

Multiple fibers 10 are folded at the proximal end 52 of the device 5 to provide several features. For example, once detached, the fibers 10 at the proximal end 52 are continuous (i.e., the free ends of each fiber are at the distal end 54 and are deployed simultaneously), which may avoid or decrease the need for meticulous placement and release of the proximal end 52. (For example, free proximal ends of single fibers deployed serially in the prior art are generally deployed well inside the coil pack so that the proximal end of the coil device doesn't migrate into the parent vessel.) Also, the continuous fibers at the proximal end transverse, or bridge, the opening (neck) of the aneurysm, thereby providing a scaffold or frame over the opening without having exposed ends. Further, the retention loop feature 30 over the folded multiple fibers 10 provides simple attachment with and detachment from the fibers to retain them in the dedicated "pusher" until released by the physician.

Detachment may be achieved by one of multiple means utilizing this primary retention loop feature 30, looped over the center 50 of these multiple fibers 10. Detachment of the retention loop 30 releases the center 50 of the multiple fibers 10. Any of several methods may be used to release the retention loop 30. In one embodiment, the loop material may extend to the proximal end of the pusher allowing the physician to release the loop directly. In another embodiment, a mechanical means translates via the pusher to the proximal end to cut or otherwise release one end of the retention loop 30.

Additionally, the mechanical properties of SMP materials can be tailored to achieve the preferred stiffness or softness for the fiber 10. Further, each fiber 10 can be fabricated from SMP materials of different formulations that result in varying stiffness along the length of the fiber, e.g., allowing a zone of loops to be stiff to anchor and the balance of the fiber to be soft for improved packing efficiency. Additionally, this material effect can be combined with diameter (dimensional) changes along the coil length to enhance achieving specific fiber stiffness in different regions along the device. Limitations found in metal wire forming inhibit this ability in other metal embolic coils.

Also, the "shape certainty" of SMP material provides the ability to accurately define and provide a straighter insertion configuration that flexes and tracks down the long, small lumen of a delivery catheter placed in the body in a tortuous path to reach the target site, and separately define and provide a deployment configuration of a complex "secondary" fiber/coil shape that enables an efficient occlusive mass. These definitive shapes and features enable using a low cost fabricated SMP device in comparison to high cost shape memory alloys (SMA) such as Nitinol, or, in comparison to traditional polymers without shape memory. Traditional polymers without shape memory undergo continuous stress in a constrained, straight packaged configuration and could not survive the shelf life and packaging duration necessary and retain appropriate post deployment shape, i.e., traditional polymer materials do not exhibit "shape certainty."

Further, SMP devices from acrylate formulations are durable implants and do not suffer from metabolic attack and degradation over time. These devices are intentionally not biodegradable but remain in the body for integration with thrombus and eventual tissue ingrowth as a permanent implant. As explained above, as SMP materials are formulated for use, other ingredients can be added to the formulation to induce specific properties or behavior that may include radio-opacity, computed tomography (CT) compatibility, tissue response, thrombogenicity, and others, or combination thereof.

In addition, rate of shape change is directly correlated to properties defined by the formulation (e.g., Tg and modulus slope at transition) relative to the temperature at which deployment occurs. Rate of shape change can be adjusted for the same deployment temperature (e.g., body core temperature) through different material formulations. This supports the ability of specific features being deployed in time before other features (e.g., anchoring) occur prior to filling.

Several exemplary design features corresponding to implementations described above are identified below. Combinations of the features discussed in this disclosure can be applied concurrently to achieve a coil device 5 with improved clinical performance. In addition to some of the features described above, other features may include the following.

Multiple Fiber Configuration: Within a given diameter of the delivery catheter, multiple coils (smaller and varying in diameter if desired) can be configured adjacent to each other resulting in a pre-deployed size that fits within the single catheter lumen. Upon deployment, the multiple coils are expressed from the catheter, change shape, and quickly generate a much more complex coil mass than could be achieved by expressing a single coil.

Individual Fiber Configuration: Each fiber works in concert with the others to achieve the total device performance. As such, each fiber is defined and formed with specific curl and arc dimensions across a specific length. For example, in one embodiment, the length of the fiber is double the length of the coil device in a pre-deployed state. Further, in some embodiments, each fiber can reflect changing diameters along its length and be fabricated with different SMP formulations to develop zones of different effective post-deployment stiffness. Zones of stiffer sections utilizing larger curls and arcs may be established to facilitate anchoring in the aneurysm and reduce any propensity of the coil pack from falling out of the aneurysm opening or neck. Likewise, zones of softer sections utilizing smaller curls and arcs are established to facilitate easy compacting such that the material fills the interior aneurysm space to very high packing factors. Zones may also be imposed that reflect differing Tg such that the shape change for each zone is tuned to occur in a specific sequence-the zones may align or differ. Each end of the individual fiber further be defined with specific curl size, material stiffness and Tg such that these ends will wrap, in some embodiments, immediately upon deployment and be retained within the aneurysm sack. In other embodiments, fibers having different diameters from each other (i.e., the diameter of each fiber is consistent along its length) may be used.

Grouped Fiber Configurations to size the aneurysm device: A specific number of individual fibers, of specific length, are grouped together that reflect the desired volume to be displaced and establish the labeled size of the coil device. The fibers are laid parallel to each other.

Detachment and sizing of the coil device 5 relative to the delivery catheter 15: In some embodiments, a retaining loop 30 (e.g., monofilament) is placed across the center 50 of the fibers 10 and the fibers are folded to form the proximal end 52 at the fold/loop (see FIGS. 3A-3E). The number of folded fibers of a certain fiber diameter establishes the size of the catheter lumen that the device can be placed into and passed for deployment. In one embodiment, the fiber's distal ends are generally even. In other embodiments, the fiber's distal ends are uneven. The loop is connected to the pusher's detachment mechanism.

Packaging, Storage, and Deployment: The fibers are heated above the highest Tg, and then placed/pulled into a constraining fixture that causes the fibers to straighten and conform to a total diameter slightly less than the associated delivery catheter size. The fibers may then be placed (before or after cooling) into a dedicated "introducer" that provides the constrained packaging for the fibers. Within this constrained package, the fibers maintain their pre-deployed shape regardless of the environmental temperature. Because the SMP material is a cross-linked polymer, the fibers maintain their shape memory ("shape certainty") through such temperature changes. Upon use, when the device in the package is at room temperature (below Tg), the elongated, pre-deployed shape presents. The device fibers are thereby easily advanced through the introducer into the proximal end of a placed delivery catheter of the physician's choice. The device/fibers are then advanced with the dedicated pusher to the aneurysm site and deployed into the sack. Once the pusher has reached the distal end of the catheter, the physician activates detachment and the device is released. The physician then determines if any additional devices or individual coils would be beneficial to achieving the highest possible packing factor.

Thus, as can be understood from the discussion found herein, the multi-fiber SMP aneurysm embolic or coiling device 5 and its various configurations as disclosed herein address current key clinical deficiencies that are unmet with existing metal and single polymer coils (and other devices and their associated challenges discussed herein). The SMP coil devices disclosed here address these clinical deficiencies by the following:
- Reducing procedure cost and time by delivering fewer devices that are more accurately sized (selected before delivery) based upon comparing measured (aneurysm) and displaced volume;
- Reducing risk due to fewer detachments from fewer devices;
- Providing a very soft fiber/coil material within the aneurysm sack that changes modulus due to the thermomechanical behavior of the SMP material, changing from a firmer material within the catheter (Stated differently, the SMP material provides sufficient stiffness to push through the catheter, yet is soft enough upon deployment due to the modulus change to fold and/or curl into a dense coil pack. A consistently soft material that does not change in modulus would likely bind in the catheter and be difficult to push down a thin catheter.);
- Providing a softer material in the aneurysm sack that enables a better packing factor and thereby increases clinical performance and clinical outcomes through reduced recurrence rates;
- Reducing cost and patient risk by allowing for more benign CT scan or MR imaging diagnostics, thus eliminating the need for angiography (contrast media and fluoroscopy) for follow-up examinations; and
- Providing all of these benefits while allowing the physician to continue to use his/her favorite delivery catheter.

All directional references (e.g., proximal, distal, upper, lower, upward, downward, left, right, lateral, front, back, top, bottom, above, below, vertical, horizontal, clockwise, and counterclockwise) are only used for identification purposes to aid the reader's understanding of the present invention, and do not create limitations, particularly as to the position, orientation, or use of the invention. Connection references (e.g., attached, coupled, connected, and joined) are to be construed broadly and may include intermediate members between a collection of elements and relative movement between elements unless otherwise indicated. As such, connection references do not necessarily infer that two elements are directly connected and in fixed relation to each other. The exemplary drawings are for purposes of illustration only and the dimensions, positions, order and relative sizes reflected in the drawings attached hereto may vary.

The above specification, examples and data provide a complete description of the structure and use of exemplary embodiments of the invention as claimed below. Although various embodiments of the invention as claimed have been described above with a certain degree of particularity, or with reference to one or more individual embodiments, those skilled in the art could make numerous alterations to the disclosed embodiments without departing from the scope of this invention. Other embodiments are therefore contemplated. It is intended that all matter contained in the above description and shown in the accompanying drawings shall be interpreted as illustrative only of particular embodiments and not limiting. Changes in detail or structure may be made without departing from the basic elements of the invention as defined in the following claims.

## Claims

1. A shape memory coiling device (5) for occluding a vessel or other vascular target comprising
a plurality of individual coiled members (10) formed in a formation state of a shape memory polymer material, wherein
in a pre-deployed state the coiled members (10) are constrained as a plurality of elongate members (10) configured for delivery through a delivery device, are attached together at a proximal end (13) of the device (5), are free with respect to each other to a distal end (14) of the device (5), and are aligned axially in parallel with respect to each other and the delivery device; and
in a deployed state the elongate members (10) revert due to a thermomechanical response to form the plurality of coiled members (10) attached at the proximal end (13) of the device (5) and separately defining complex coiled shapes to form a dense coil pack to fill the vascular target; and
in an intra-deployed state between the pre-deployed state and the deployed state, at least one of the plurality of elongate members (10) is configured to anchor the coiling device (5) while filling the vascular target and reverting in form to a coiled member (10).

2. The coiling device of claim 1, wherein
the plurality of elongate members (10) is folded at an intermediate point of each of the plurality of elongate members; and/or
the plurality of elongate members (10) is connected together at an intermediate point of each of the plurality of elongate members.

3. The coiling device of claim 1, wherein
the plurality of coiled members (10) includes members having different curl and arc shapes and sizes with respect to each other; and/or
two or more of the coiled members have varying diameters with respect to each other; and/or
one or more of the coiled members is helical in shape.

4. The coiling device of claim 1, wherein at least one of the plurality of coiled members comprises at least two zones of material with different material properties selected from the group comprising a modulus and a glass transition temperature (Tg).

5. The coiling device of claim 1, wherein
each of two or more of the plurality of coiled members comprises at least two zones of material with different material properties selected from the group comprising a modulus and a glass transition temperature (Tg); and
the at least two zones of material are aligned relative to each individual elongate member; or
the at least two zones of material are not aligned relative to each individual elongate member.

6. The coiling device of claim 1, wherein
each of the plurality of elongate members has a common length; or
two or more of the plurality of elongate members have a different length.

7. A method of manufacturing a shape memory coiling device (5) for occluding a vascular target comprising
providing a shape memory polymer material;
forming a plurality of individual coiled members (10) from the shape memory polymer material in a formation state;
forming at least one of the individual coiled members (10) in a configuration to anchor the coiling device (5) while filling the vascular target with the individual coiled members (10);
attaching the plurality of individual coiled members (10) together at a proximal end (13) of the device (5) while leaving the coiled members (10) free with respect to each other to the distal end (14) of the device;
constraining the plurality of coiled members (10) in a pre-deployed state as a plurality of elongate members (10); and
arranging the plurality of elongate members (10) axially in parallel with respect to each other.

8. The method of claim 7, further comprising
forming one or more of the plurality of coiled members (10) as a helical shape in the formation state; and/or
forming two or more of the plurality of coiled members (10) with varying diameters with respect to each other in the formation state; and/or
forming members the plurality of coiled members with different curl and arc shapes and sizes with respect to each other in the formation state.

9. The method of claim 7, wherein each of the plurality of coiled members (10) has at least two zones of shape memory polymer material each zone having a different modulus or a different glass transition temperature (Tg), or both.

10. The method of claim 7 wherein each of the plurality of elongate members has a proximal end (13) and a distal end (14) and the step of attaching further comprises connecting the proximal ends of each of the plurality of elongate members together.

11. The method of claim 10 further comprising
providing a delivery device (20) having a pusher mechanism with a detachment feature (30); and
operably attaching the connected proximal ends of the plurality of elongate members to the detachment feature (30) of the pusher mechanism of the delivery device (20).

12. The method of claim 7 further comprising
providing a retaining loop across an intermediate point of each individual member of the plurality of elongate members; and
folding the plurality of elongate members about the retaining loop to form a proximal end (13) at the retaining loop and a distal end (54) of the plurality of elongate members at adjacent free ends of the plurality of elongate members.

13. The method of claim 12 further comprising
providing a delivery device (20) having a pusher mechanism with a detachment feature (30); and
operably attaching the proximal end (13) to the detachment feature (30) of the pusher mechanism of the delivery device.

## Patentansprüche

1. Formspeichernde Coiling-Vorrichtung (5) zum Verschließen eines Gefäßes oder eines anderen vaskulären Targets, umfassend:
eine Vielzahl von einzelnen gewundenen Elementen (10), die in einem Formierungszustand eines formspeichernden Polymermaterials gebildet wurden, wobei
die gewundenen Elemente (10) in einem Zustand vor der Auslösung als Vielzahl von länglichen Elementen (10) zusammengehalten werden, die zur Abgabe durch eine Abgabevorrichtung ausgelegt sind, an einem proximalen Ende (13) der Vorrichtung (5) aneinander angebracht sind, an einem distalen Ende (14) der Vorrichtung (5) frei voneinander und achsparallel zueinander und zur Abgabevorrichtung ausgerichtet sind; und
die länglichen Elemente (10) in einem ausgelösten Zustand aufgrund einer thermomechanischen Reaktion zur Bildung der Vielzahl von gewundenen Elementen (10) am proximalen Ende (13) der Vorrichtung (5) zurückkehren und getrennt komplexe gewundene Formen definieren, um ein dichtes Spulenpaket zum Füllen des Targets zu bilden; und
in einem Zustand während der Auslösung zwischen dem Zustand vor der Auslösung und dem ausgelösten Zustand mindestens eines der Vielzahl von länglichen Elementen (10) zum Verankern der Coiling-Vorrichtung (5) beim Füllen des vaskulären Targets und Zurückkehren zur Form eines gewundenen Elements (10) ausgelegt ist.

2. Coiling-Vorrichtung nach Anspruch 1, wobei
die Vielzahl von länglichen Elementen (10) an einem Zwischenpunkt eines jeden der Vielzahl von länglichen Elementen zusammengefaltet ist und/oder
die Vielzahl von länglichen Elementen (10) an einem Zwischenpunkt eines jeden der Vielzahl von länglichen Elementen miteinander verbunden ist.

3. Coiling-Vorrichtung nach Anspruch 1, wobei
die Vielzahl von gewundenen Elementen (10) Elemente beinhaltet, die untereinander verschiedene Windungen sowie Bogenformen und -größen aufweisen; und/oder
zwei oder mehr der gewundenen Elemente voneinander abweichende Durchmesser haben; und/oder
eines oder mehrere der gewundenen Elemente spiralförmig ist/sind.

4. Coiling-Vorrichtung nach Anspruch 1, wobei mindestens eines der Vielzahl von gewundenen Elementen mindestens zwei Materialzonen mit unterschiedlichen Materialeigenschaften, die aus der Gruppe umfassend einen Modul und eine Glasübergangstemperatur (Tg) ausgewählt sind, umfasst.

5. Coiling-Vorrichtung nach Anspruch 1, wobei
jedes von zwei oder mehr der Vielzahl von gewundenen Elementen mindestens zwei Materialzonen mit unterschiedlichen Materialeigenschaften, die aus der Gruppe umfassend einen Modul und eine Glasübergangstemperatur (Tg) ausgewählt sind, umfasst; und
die mindestens zwei Materialzonen an jedem einzelnen länglichen Element ausgerichtet sind; oder
die mindestens zwei Materialzonen nicht an jedem einzelnen länglichen Element ausgerichtet sind.

6. Coiling-Vorrichtung nach Anspruch 1, wobei
jedes der Vielzahl von länglichen Elementen eine gleiche Länge hat; oder
zwei oder mehrere der Vielzahl von länglichen Elementen eine unterschiedliche Länge haben.

7. Verfahren zur Herstellung einer formspeichernden Coiling-Vorrichtung (5) zum Verschließen eines vaskulären Targets, umfassend:
Bereitstellen eines formspeichernden Polymermaterials;
Ausbilden einer Vielzahl von einzelnen gewundenen Elementen (10) aus dem formspeichernden Polymermaterial in einem Formierungszustand;
Ausbilden mindestens eines der einzelnen gewundenen Elemente (10) in einer Konfiguration zum Verankern der Coiling-Vorrichtung (5) beim Füllen des vaskulären Targets mit den einzelnen gewundenen Elementen (10);
Anbringen der Vielzahl von einzelnen gewundenen Elementen (10) aneinander an einem proximalen Ende (13) der Vorrichtung (5), während die gewundenen Elemente (10) am distalen Ende (14) der Vorrichtung frei voneinander belassen werden;
Zusammenhalten der Vielzahl von gewundenen Elementen (10) in einem Zustand vor der Auslösung als Vielzahl von länglichen Elementen (10); und
Anordnen der Vielzahl von länglichen Elementen (10) achsparallel zueinander.

8. Verfahren nach Anspruch 7, ferner umfassend:
Ausbilden eines oder mehrerer der Vielzahl von gewundenen Elementen (10) als Spiralform im Formierungszustand; und/oder
Ausbilden von zwei oder mehreren der Vielzahl von gewundenen Elementen (10) mit voneinander verschiedenem Durchmesser im Formierungszustand; und/oder
Ausbilden von Elementen der Vielzahl von gewundenen Elementen mit untereinander verschiedenen Windungen und Bogenformen und -größen im Formierungszustand.

9. Verfahren nach Anspruch 7, wobei jedes der Vielzahl von gewundenen Elementen (10) mindestens zwei Zonen von formspeicherndem Polymermaterial aufweist, wobei jede Zone einen anderen Modul oder eine andere Glasübergangstemperatur (Tg) oder beides aufweist.

10. Verfahren nach Anspruch 7, wobei jedes der Vielzahl von länglichen Elementen ein proximales Ende (13) und ein distales Ende (14) aufweist und der Schritt des Anbringens ferner ein Verbinden der proximalen Enden eines jeden der Vielzahl von länglichen Elementen miteinander umfasst.

11. Verfahren nach Anspruch 10, ferner umfassend:
Bereitstellen einer Abgabevorrichtung (20), die einen Schubmechanismus mit einer Abtrennfunktion (30) aufweist; und
betriebsfähiges Anbringen der miteinander verbundenen proximalen Enden der Vielzahl von länglichen Elementen an der Abtrennfunktion (30) des Schubmechanismus der Abgabevorrichtung (20).

12. Verfahren nach Anspruch 7, weiterhin umfassend:
Bereitstellen einer Halteschlaufe über einen Zwischenpunkt jedes einzelnen Elements der Vielzahl von länglichen Elementen; und
Falten der Vielzahl von länglichen Elementen um die Halteschlaufe zur Bildung eines proximalen Endes (13) an der Halteschlaufe und eines distalen Endes (54) der Vielzahl von länglichen Elementen an benachbarten freien Enden der Vielzahl von länglichen Elementen.

13. Verfahren nach Anspruch 12, ferner umfassend:
Bereitstellen einer Abgabevorrichtung (20), die einen Schubmechanismus mit einer Abtrennfunktion (30) aufweist; und
betriebsfähiges Anbringen des proximalen Endes (13) an der Abtrennfunktion (30) des Schubmechanismus der Abgabevorrichtung.

## Revendications

1. Dispositif d'enroulement à mémoire de forme (5) pour obturer un vaisseau ou une autre cible vasculaire comprenant
une pluralité d'éléments enroulés individuels (10) formée dans un état de formation d'un matériau polymère à mémoire de forme, dans lequel
dans un état pré-déployé, les éléments enroulés (10) sont construits sous forme d'une pluralité d'éléments allongés (10) conçue pour l'administration à travers un dispositif d'administration, sont liés ensemble à une extrémité proximale (13) du dispositif (5), sont libres les uns par rapport aux autres à une extrémité distale (14) du dispositif (5), et sont alignés axialement en parallèle les uns par rapport aux autres et au dispositif d'administration ; et
dans un état déployé les éléments allongés (10) reprennent leur forme en raison d'une réponse thermomécanique pour former la pluralité des éléments enroulés (10) liée à l'extrémité proximale (13) du dispositif (5) et définissant séparément des formes enroulées complexes pour former un paquet enroulé dense pour remplir la cible vasculaire ; et
dans un état intra-déployé entre l'état pré-déployé et l'état déployé, au moins un de la pluralité des éléments allongés (10) est conçu pour ancrer le dispositif d'enroulement (5) tout en remplissant la cible vasculaire et en reprenant la forme d'un élément enroulé (10).

2. Dispositif d'enroulement de la revendication 1, dans lequel
la pluralité des éléments allongés (10) est pliée au niveau d'un point intermédiaire de chacun de la pluralité des éléments allongés ; et/ou
la pluralité des éléments allongés (10) est connectée ensemble au niveau d'un point intermédiaire de chacun de la pluralité des éléments allongés.

3. Dispositif d'enroulement de la revendication 1, dans lequel
la pluralité des éléments enroulés (10) comprend des éléments ayant différentes formes et tailles de boucle et d'arc les uns par rapport aux autres ; et/ou
deux ou plusieurs des éléments enroulés ont des diamètres variables les uns par rapport aux autres ; et/ou
un ou plusieurs des éléments enroulés a une forme d'hélice.

4. Dispositif d'enroulement de la revendication 1, dans lequel au moins un de la pluralité des éléments enroulés comprend au moins deux zones de matériau ayant des propriétés matérielles différentes choisies dans le groupe composé d'un module et d'une température de transition vitreuse (Tg).

5. Dispositif d'enroulement de la revendication 1, dans lequel
chacun des deux ou des plusieurs de la pluralité des éléments enroulés comprennent au moins deux zones de matériau ayant des propriétés matérielles différentes choisies dans le groupe composé d'un module et d'une température de transition vitreuse (Tg) ; et
l'au moins deux zones de matériau sont alignées par rapport à chaque élément allongé individuel; ou
l'au moins deux zones de matériau ne sont pas alignées par rapport à chaque élément allongé individuel.

6. Dispositif d'enroulement de la revendication 1, dans lequel
Chacun de la pluralité des éléments allongés a une longueur commune ; ou
deux ou plusieurs de la pluralité des éléments allongés a une longueur différente.

7. Procédé de fabrication d'un dispositif d'enroulement à mémoire de forme (5) pour obturer une cible vasculaire comprenant
l'utilisation d'un matériau polymère à mémoire de forme ;
la formation d'une pluralité d'éléments enroulés individuels (10) à partir du matériau polymère à mémoire de forme dans un état de formation ;
la formation d'au moins un des éléments enroulés individuels (10) dans une configuration pour ancrer le dispositif d'enroulement (5) tout en remplissant la cible vasculaire avec des éléments enroulés individuels (10) ;
la fixation de la pluralité des éléments enroulés individuels (10) ensemble au niveau d'une extrémité proximale (13) du dispositif (5) tout en laissant les éléments enroulés (10) libres les uns par rapport aux autres au niveau de l'extrémité distale (14) du dispositif ;
le fait de contraindre la pluralité des éléments enroulés (10) dans un état pré-déployé sous forme d'une pluralité d'éléments allongés (10) ; et
la disposition de la pluralité d'éléments allongés (10) axialement en parallèle les uns par rapport aux autres.

8. Procédé de la revendication 7, comprenant également
la formation d'un ou des plusieurs de la pluralité d'éléments allongés (10) en une forme d'hélice dans l'état de formation ; et/ou
la formation de deux ou de plusieurs de la pluralité d'éléments allongés (10) avec des diamètres variables les uns par rapport aux autres dans l'état de formation ; et/ou
la formation des éléments de la pluralité d'éléments allongés ayant différentes formes et tailles de boucle et d'arc les uns par rapport aux autres dans l'état de formation.

9. Procédé de la revendication 7, dans lequel chacun de la pluralité des éléments enroulés (10) comporte au moins deux zones de matériau polymère à mémoire de forme chaque zone ayant un module différent et une température de transition vitreuse différente (Tg), ou les deux.

10. Procédé de la revendication 7, dans lequel chacun de la pluralité des éléments allongés a une extrémité proximale (13) et une extrémité distale (14) et l'étape de fixation comprend également le rattachement des extrémités proximales de chacun de la pluralité des éléments allongés ensemble.

11. Procédé de la revendication 10, comprenant également
l'utilisation d'un dispositif d'administration (20) muni d'un mécanisme poussoir avec un élément de libération (30) ; et
qui rattache en fonctionnement les extrémités proximales rattachées de la pluralité des éléments allongés à l'élément de libration (30) du mécanisme poussoir du dispositif d'administration (20).

12. Procédé de la revendication 7, comprenant également
la fourniture d'une boucle de retenue à travers un point intermédiaire de chaque élément individuel de la pluralité des éléments allongés ; et
le repliement de la pluralité des éléments allongés autour de la boucle de retenue pour former une extrémité proximale (13) au niveau de la boucle de retenue et une extrémité distale (54) de la pluralité des éléments allongés au niveau des extrémités libres adjacentes de la pluralité des éléments allongés.

13. Procédé de la revendication 12, comprenant également
l'utilisation d'un dispositif d'administration (20) muni d'un mécanisme poussoir avec un élément de libération (30) ; et
le rattachement en fonctionnement de l'extrémité proximale (13) à l'élément de libération (30) du mécanisme poussoir du dispositif d'administration.
